# EUROPÄISCHE PATENTSCHRIFT

(11) **EP 0 534 314 B1**
(45) Veröffentlichungstag und Bekanntmachung des Hinweises auf die Patenterteilung: **05.06.1996**
(21) Anmeldenummer: 92115905.9
(22) Anmeldetag: 17.09.1992
(51) Int. Cl.: C07C 205/45, C07C 201/12, C09B 29/42, C09B 67/48, C09B 67/00, C09B 29/36

(54) **Verfahren zur Herstellung von Nitrobenzophenonen sowie färbestabile Modifikation eines Benzophenonazopyridonfarbstoffs**
Process for the preparation of nitrobenzophenones and also the colour-fast modification of a benzophenoneazopyridone dye
Procédé de préparation de nitrobenzophénones ainsi que la modification stable d'un colorant benzophénoneazopyridone

(30) Priorität: 25.09.1991 DE 4131844
(43) Veröffentlichungstag der Anmeldung: 31.03.1993
(73) Patentinhaber: BASF Aktiengesellschaft, D-67063 Ludwigshafen (DE)
(72) Erfinder: Degen, Helmut, W-6710 Frankenthal (DE); Zimmermann, Norbert, W-6701 Waldsee (DE); Brueckmann, Ralf, Dr., Charlotte, N.C. 28270 (US); Lamm, Gunther, Dr., W-6733 Hassloch (DE); Lange, Arno, Dr., W-6702 Bad Duerkheim (DE); Reichelt, Helmut, Dr., W-6730 Neustadt (DE)

(56) Entgegenhaltungen:
- EP-A- 0 068 186
- EP-A- 0 093 590
- FR-A- 2 076 138
- FR-A- 2 160 583
- FR-A- 2 170 223
- US-A- 2 007 382
- CHEMICAL ABSTRACTS, Band 101, Nr. 18, 29. Oktober 1984, Seite 79, Columbus, Ohio, US; Zusammenfassung Nr. 153476p; & JP-A-5 996 168
- JOURNAL OF ORGANIC CHEMISTRY, Band 54, 1989, Seiten 3478-3482, Easton, US; M.A. FINDEIS et al: "Nitrobenzophenone oxime based resins for the solid-phase synthesis of protected peptide segments"
- CHEMICAL ABSTRACTS, Band 84, 1976, Seite 549, Spalte 2, Zusammenfassung Nr. 105291, Columbus, Ohio, US; & SU-A-430 631

## Beschreibung

Die vorliegende Erfindung betrifft ein neues Verfahren zur Herstellung von Nitrobenzophenonen durch Umsetzung von Benzol oder dessen Derivaten mit Nitrobenzoylchlorid in Gegenwart einer Lewis-Säure, eine färbestabile Modifikation von 1,4-Dimethyl-5-cyano-3-[4-(2,4-dimethylbenzoyl)phenylazo]-2-hydroxypyrid-6-on sowie dessen Verwendung zum Färben oder Bedrucken von synthetischem Fasermaterial.

Die Herstellung von Nitrobenzophenonen durch Umsetzung von Nitrobenzoylchlorid mit Benzol oder dessen Derivaten in einer Friedel-Crafts-Reaktion ist bekannt. Beispielsweise wird in der EP-A-66153 die Synthese von 4-Nitro-4'-isopropylbenzophenon beschrieben, wobei als inertes Lösungsmittel Chlorbenzol oder Dichlorethan zur Anwendung gelangt. Diese Lösungsmittel, wie auch andere, z.B. Nitrobenzol, die üblicherweise bei diesem Reaktionstyp zur Anwendung gelangen, belasten jedoch das Abwasser.

In FR-A-21 70 223 wird in Beispiel 5 die Synthese von p-Nitro-benzophenon aus p-Nitrobenzoylchlorid und Benzol unter Eisen(III)-chlorid-Katalyse und in Abwesenheit von Lösungsmittel beschrieben. Mit dem Ausgangsstoff Benzol ist die Ausbeute allerdings unbefriedigend niedrig.

Weiterhin wird in der SU-A-430 631 die Herstellung von 4-Nitro-2',4'-dimethylbenzophenon beschrieben. Man setzt dort p-Nitrobenzoylchlorid mit einem 3-molaren Überschuß an m-Xylol in Gegenwart von Eisenchlorid bei einer Temperatur von 150°C um. Die Ausbeute an Benzophenon ist jedoch unbefriedigend.

Aus der DE-A-2 201 208 ist schließlich ein Verfahren bekannt, in dem p-Nitrobenzoylchlorid mit m-Xylol oder Anisol jeweils in Abwesenheit von inertem organischem Lösungsmittel in Gegenwart von 2,4,6-Trinitrobenzolsulfonsäure als Katalysator zur Reaktion gebracht wird. Das Molverhältnis Benzolderivat : p-Nitrobenzoylchlorid beträgt dabei 1,5 : 1. Nachteilig an diesem Verfahren ist aber die hohe Reaktionstemperatur sowie die unbefriedigende Ausbeute an Zielprodukt.

Aufgabe der vorliegenden Erfindung war es nun, ein neues Verfahren zur Herstellung von Nitrobenzophenonen bereitzustellen, das auf einer Friedel-Crafts-Acylierung beruht, bei dem die obengenannten Nachteile aber vermieden werden. Das neue Verfahren sollte außerdem technisch einfach durchzuführen sein und die Zielprodukte in hoher Ausbeute und Reinheit liefern.

Es wurde nun gefunden, daß die Herstellung von Nitrobenzophenonen der Formel I 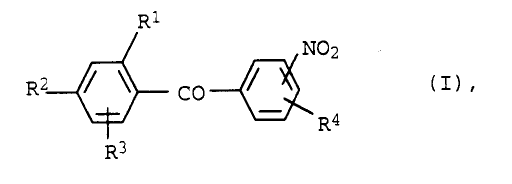
in der R¹ und R² gleich oder verschieden sind und unabhängig voneinander jeweils C₁-C₄-Alkyl, R³ Wasserstoff und R⁴ Wasserstoff oder Halogen bedeuten, durch Umsetzung von Aromaten der Formel II 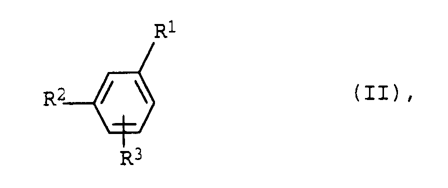
in der R¹, R² und R³ jeweils die obengenannte Bedeutung besitzen, mit Nitrobenzoylchloriden der Formel III 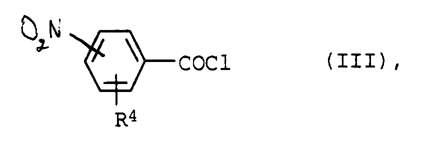
in der R⁴ die obengenannte Bedeutung besitzt, in Gegenwart einer Lewis-Säure vorteilhaft gelingt, wenn man die Umsetzung im wesentlichen in Abwesenheit von inerten Lösungsmitteln bei einer Temperatur von 50 bis 110°C und einem Molverhältnis von Aromat II : Nitrobenzoylchlorid III von 1 : 1 bis 2 : 1 durchführt und als Lewis-Säure katalytische Mengen an Eisen(III)chlorid verwendet.

Reste R¹ und R² in Formel I sind z.B. Methyl, Ethyl, Propyl, Isopropyl, Butyl, Isobutyl, sec-Butyl, tert-Butyl.

Wenn R⁴ Halogen bedeutet, kommt z.B. Chlor oder Brom in Betracht.

Für das erfindungsgemäße Verfahren geeignete Aromaten der Formel II sind z.B. o-, m- oder p-Xylol, 3-Ethyltoluol, m-Diisopropylbenzol, m-Diisobutylbenzol.

Hervorzuheben ist eine Verfahrensweise, in der ein Aromat der Formel IIa 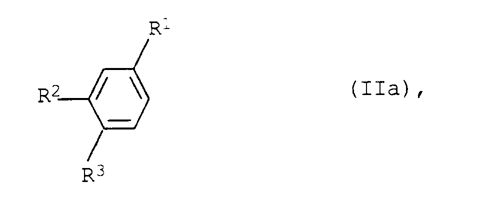
in der R¹, R² und R³ jeweils die obengenannte Bedeutung besitzen, als Ausgangsprodukt verwendet wird.

Bevorzugt ist eine Verfahrensweise, in der in Formel II R¹ und R² jeweils Methyl und R³ Wasserstoff bedeuten.

Als Nitrobenzoylchloride III kommen z.B. o-, m- oder p-Nitrobenzoylchlorid oder 3-Nitro-4-chlorbenzoylchlorid in Betracht, wobei die Verwendung von m- oder p-Nitrobenzoylchlorid bevorzugt ist. Besonders hervorzuheben ist die Verwendung von p-Nitrobenzoylchlorid.

Als Lewis-Säure wird erfindungsgemäß Eisen(III)chlorid verwendet. Es ist aber auch möglich, Eisen(III)oxid anzuwenden, dies wird durch den bei der Umsetzungen entstehenden Chlorwasserstoff in Eisen(III)chlorid übergeführt.

Das Molverhältnis von Aromat II : Nitrobenzoylchlorid betragt in der Regel 1 : 1 bis 2 : 1, vorzugsweise 1,1 : 1 bis 1,3 : 1.

Die Zugabe der Lewis-Säure erfolgt, wie oben bereits ausgeführt, in katalytischen Mengen. Darunter können z.B. 0,5 bis 20 Gew.%, vorzugsweise 2 bis 8 Gew.% Lewis-Saure, jeweils bezogen auf den Aromat II, verstanden werden.

Das erfindungsgemäße Verfahren wird im wesentlichen in Abwesenheit von inerten Lösungsmitteln durchgeführt, d.h. in der Regel wird es in vollständiger Abwesenheit von inerten Lösungsmitteln vorgenommen, wobei es jedoch auch möglich ist, in Anwesenheit von bis zu 10 Gew.-%, vorzugsweise bis zu 5 Gew.-%, jeweils bezogen auf das Gewicht an Aromat II, eines inerten Lösungsmittels zu arbeiten. Geeignete inerte Lösungsmittel sind z.B. Chlorbenzol, Dichlorbenzol oder Nitrobenzol.

Das erfindungsgemäße Verfahren wird zweckmäßig so durchgeführt, daß man ein Gemisch von Aromat II, Nitrobenzoylchlorid III und Lewis-Säure bereitet und dieses unter Rühren auf eine Temperatur von 50 bis 110°C, vorzugsweise 50 bis 90°C erhitzt. Anschließend erfolgt eine Nachrührphase, die bei der obengenannten Temperatur durchgeführt wird und im allgemeinen 3 bis 6 Stunden in Anspruch nimmt.

Während der gesamten Reaktionsdauer wird der bei der Acylierungsreaktion gebildete Chlorwasserstoff aus dem Reaktionsgemisch abgeleitet.

Nach beendeter Umsetzung kann gegebenenfalls überschüssiger Aromat der Formel II mittels Wasserdampfdestillation entfernt und zurückgewonnen werden. Das resultierende Nitrobenzophenon der Formel I kann direkt für Folgereaktionen verwendet werden.

In einer besonders vorteilhaften Variante geht man von Nitrobenzoesäure aus und erzeugt Nitrobenzoylchlorid in situ. Die Nitrobenzoesäure wird dabei im obengenannten Molverhältnis (Aromat II : Nitrobenzoylchlorid III) im Aromat II suspendiert. Gegebenenfalls durch die Säure eingeschleppte Mengen an Wasser können mittels azeotroper Destillation entfernt werden.

Zur resultierenden Suspension gibt man bei einer Temperatur von 60 bis 100°C, gegebenenfalls in Anwesenheit von katalytischen Mengen an N,N-Dimethylformamid, Pyridin oder 4-Dimethylaminopyridin, 1 bis 1,1 mol Thionylchlorid, bezogen auf 1 Mol Nitrobenzoesäure, und erwärmt das Reaktionsgemisch auf eine Temperatur von 70 bis 100°C. Nach einer Reaktionsdauer von 3 bis 6 Stunden ist das Nitrobenzoylchlorid gebildet und gegebenenfalls überschüssiges Thionylchlorid kann abdestilliert werden. Danach erfolgt durch die Zugabe von katalytischen Mengen an Lewis-Säure, wie oben dargelegt, die Friedel-Crafts-Acylierung.

Im erfindungsgemäßen Verfahren, das sowohl kontinuierlich als auch diskontinuierlich vorgenommen werden kann, kann auf die Verwendung von inerten Lösungsmitteln verzichtet werden. Gleichzeitig ist das neue Verfahren sehr einfach durchzuführen und die Zielprodukte fallen in hoher Ausbeute und Reinheit an.

Bei den Nitrobenzophenonen der Formel I handelt es sich um wertvolle Zwischenprodukte für die Herstellung von Azofarbstoffen.

Indem man die Nitrobenzophenone I nach an sich bekannten Methoden, z.B. mittels katalytischer Hydrierung, in die entsprechenden Aminobenzophenone der Formel Ia 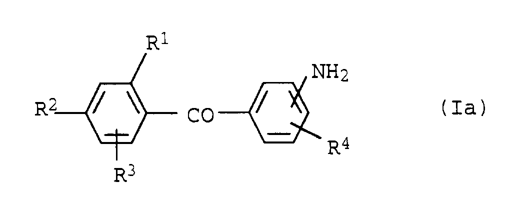
überführt, in der R¹, R², R³ und R⁴ jeweils die obengenannte Bedeutung besitzen, gelangt man zu wertvollen Diazokomponenten für die Herstellung von Azofarbstoffen wie sie z.B. aus der DE-A-2 001 821 oder DE-A-2 157 229 bekannt sind.

Die vorliegende Erfindung betrifft weiterhin eine färbestabile Modifikation des Farbstoffs der Formel IV 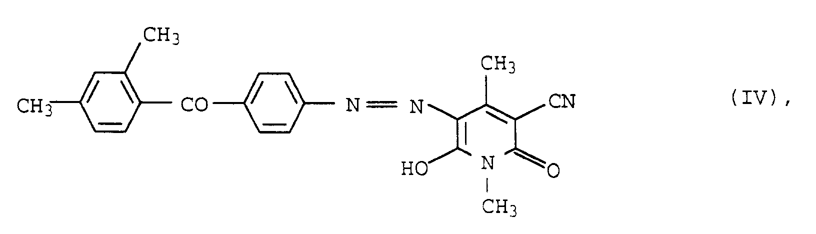
mit dem Röntgenbeugungsdiagramm (Cu-K_{α}-Strahlung) mit folgenden Linien starker Intensität Beugungswinkel Θ [°] von 8,085, 12,772, 13,644, 24,864 und 25,656, Linien mittlerer Intensität bei Beugungswinkel Θ [°] von 16,402, 18,894, 20,822, 21,940, 27,546 und 28,256 sowie Linien schwacher Intensität bei Beugungswinkel Θ [°] von 14,490, 15,352 und 23,711.

Die färbeinstabile Modifikation des Farbstoffs der Formel IV weist ein Röntgenbeugungsdiagramm (Cu-K_{α}-Strahlung) mit folgenden Linien starker Intensität bei Beugungswinkel Θ [°] von 8,407, 12,821 und 25,231, Linien mittlerer Intensität bei Beugungswinkel Θ [°] von 13,822, 21,921, 25,865 und 26,547 sowie Linien geringer Intensität bei Beugungswinkel Θ [°] von 15,404, 16,641 und 27,579, auf.

Für die Aufnahme der Röntgenbeugungsdiagramme wurde jeweils ein Pulverdiffraktometer der Firma Siemens vom Typ D 5000 verwendet.

Der Farbstoff der Formel IV ist aus der DE-A-2 157 229 bekannt. Ein Herstellverfahren ist dort nicht beschrieben. Erzeugt man jedoch den Farbstoff nach der in der DE-A-2 001 821 genannten Methode, nämlich durch Diazotieren von 4-Amino-2',4'-dimethylbenzophenon in 30 gew.%iger Salzsäure mit Natriumnitrit und anschließendes Kuppeln mit 1,4-Dimethyl-3-cyano-2-hydroxypyrid-6-on, fällt eine Modifikation an, die unter den in der Färbepraxis auftretenden Bedingungen keine genügende Stabilität besitzt.

Die färbeinstabile Modifikation des Farbstoffs IV kann durch halb- bis vierstündiges Behandeln in wäßriger Lauge bei einem pH-Wert von 7 bis 9 bei einer Temperatur von 20 bis 90°C in die färbestabile Modifikation umgewandelt werden.

Es ist auch möglich, die färbestabile Modifikation direkt bei der Synthese des Farbstoffs zu erzeugen. Dies kann durch Durchführung der Kupplungsreaktion (Umsetzung des Diazoniumsalzes von 4-Amino-2',4'-dimethylbenzophenon mit 1,4-Dimethyl-5-cyano-2-hydroxypyrid-6-on) bei einem pH-Wert von 7 bis 10,5 erreicht werden.

Die neue färbestabile Modifikation des Farbstoffs der Formel IV eignet sich in vorteilhafter Weise zum Färben oder Bedrucken von synthetischem Fasermaterial, beispielsweise von Polyestergewebe.

Man erhält Färbungen in gelbem Farbton, die sich durch hohe Farbstärke, hohen Aufziehgrad sowie sehr gute Licht- und Thermofixierechtheit auszeichnen.

Die folgenden Beispiele sollen die Erfindung näher erläutern.

### Beispiel 1

a) 210 ml m-Xylol wurden mit 167 g p-Nitrobenzoesäure versetzt. Geringe Mengen an Wasser, die durch die p-Nitrobenzoesäure eingeschleppt wurden, kreiste man unter Erhitzen auf Siedetemperatur mit Hilfe eines Wasserabscheiders aus. Anschließend kühlte man auf 70°C ab, gab 1 g N,N-Dimethylformamid hinzu und tropfte dann bei 70 bis 75°C 88 ml Thionylchlorid hinzu. Man rührte 3 Stunden bei 80 bis 85°C nach und erhielt so eine Lösung von p-Nitrobenzoylchlorid in m-Xylol. Diese Lösung wurde durch Abdestillieren einer kleinen Menge an m-Xylol frei von Thionylchlorid gemacht.
b) (nicht erfindungsgemäß)
   Dann kühlte man auf Raumtemperatur ab und setzte 17 g wasserfreies Aluminiumchloridpulver hinzu. Man erhitzte innerhalb von 1,5 Stunden auf 100 bis 110°C, wobei Chlorwasserstoffentwicklung auftrat, und rührte 3 Stunden bei dieser Temperatur nach, bis die Chlorwasserstoffentwicklung beendet war. Man kühlte das Gemisch auf 90°C ab und zerlegte es auf 600 ml Wasser. Danach destillierte man überschüssiges m-Xylol mit Wasserdampf ab, versetzte den Rückstand mit 0,5 g eines sauer wirksamen Netzmittels und kühlte unter Rühren auf Raumtemperatur ab. Nach Absaugen, Waschen mit Wasser und Trocknen erhielt man 255 g des Benzophenons der Formel 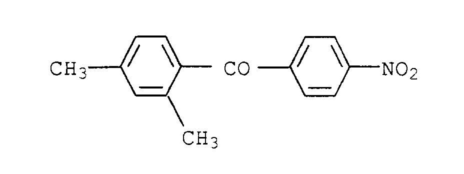 H¹NMR (DMSO-D₆) [ppm]
   8,35 (d 2)
   7,99 (d 2)
   7,28
   7,25
   7,14
   2,38 (s 3)
   2,30 (s 3)
   Das zurückgewonnene m-Xylol war einheitlich. Es kann für weitere Umsetzungen wieder verwendet werden.
c) Das unter b) erhaltene 4-Nitro-2',4'-dimethyl-benzophenon wurde mit 900 ml Isobutanol und 3,5 g aktiviertem Nickelpulver versetzt und bei 50 bis 60°C bei geringem Überdruck (1,25 bar) mit Wasserstoff zu 4-Amino-2',4'-dimethylbenzophenon hydriert. Nach Abfiltrieren des Katalysators destillierte man das organische Lösungsmittel zur Wiedergewinnung ab, versetzte den Rückstand bei 100 bis 110°C mit Wasser, das eine Temperatur von 95°C aufwies, und rührte nach bis Raumtemperatur erreicht war. 4-Amino-2',4'-dimethylbenzophenon kristallisierte dabei bei 90°C aus. Das kristalline Produkt wurde abgesaugt, mit Wasser gewaschen und getrocknet. Man erhielt 215 g an Aminoverbindung (Fp. 121°C).
   H¹-NMR (DMSO-D₆) [ppm]:
   7,40 (d 2)
   7,15 (s 1)
   6,97 (s 1)
   6,55 (d 2)
   6,21 (s 2 - NH₂)
   2,29 (s 3 - CH₃)
   2,09 (s 3 - CH₃)
d) 226 g 4-Amino-2',4'-dimethylbenzophenon wurden bei 50 bis 60°C in 350 ml 27 gew.%iger Schwefelsäure gelöst. Dann gab man 450 ml 5N-Salzsäure und 2 g eines sauer wirksamen Netzmittels hinzu und rührte das Gemisch kalt, wodurch die Aminokomponente als Hydrochlorid kristallin gefällt wurde. Nun gab man bei 0 bis 7°C 320 ml 23 gew.°ige wäßrige Natriumnitritlösung hinzu und rührte die entstehende trübe Diazoniumsalzlösung 2,5 Stunden bei 0 bis 5°C nach. Dann wurde überschüssige salpetrige Säure mit Amidosulfonsäure zerstört. Die Reaktionsmischung gab man zu einem Gemisch, das wie folgt hergestellt wurde:
   164 g 1,4-Dimethyl-5-cyano-2-hydroxypyrid-6-on wurden in 2000 ml Wasser und Natronlauge bei pH 7,5 gelöst. Die Lösung wurde durch Zugabe von Eis auf 0°C abgekühlt und mit 20 g Soda versetzt.
   Während der Zugabe des Diazoniumsalzes gab man gleichzeitig verdünnte Natronlauge zu, so daß der pH-Wert des Reaktionsgemisches im Bereich von 6 bis 9 gehalten wurde. Die Kupplung war nach Zugabe des Diazoniumsalzes rasch beendet. Man stellte den pH-Wert auf 7,5 bis 8,0, rührte 1,5 Stunden nach und erhitzte das Gemisch bei diesem pH-Wert auf 85 bis 95°C und temperte 45 Minuten. Danach wurde die Farbstoffsuspension abfiltriert, das Nutschgut mit Wasser salzfrei gewaschen und getrocknet.
   Der resultierende Farbstoff der Formel 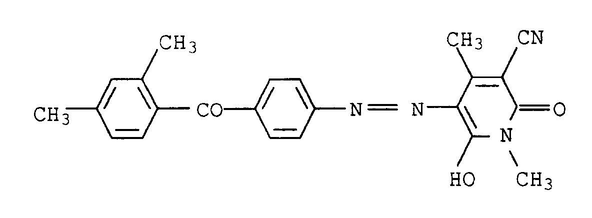 schmilzt bei 183°C.
   Er zeichnet sich durch folgendes Röntgenspektrum aus: Beugungswinkel Θ [°] von 8,085, 12,772, 13,644, 24,864 und 25,656, Linien mittlerer Intensität bei Beugungswinkel Θ [°] von 16,402, 18,894, 20,822, 21,940, 27,546 und 28,256 sowie Linien schwacher Intensität bei Beugungswinkel Θ [°] von 14,490, 15,352 und 23,711.
e) 45 g des unter d) erhaltenen Farbstoffs wurden mit 45 g eines Dispergiermittels auf Basis eines Naphthalinsulfonsäure-Formaldehyd-Kondensats, das in der EP-A-463 401 als Dispergiermittel 3 beschrieben ist, angeteigt und mit Wasser auf einen Feststoffgehalt von 35 Gew.% gestellt und bei einem pH-Wert von 8,5 in einer Sandmühle bis zur Erreichung einer guten Feinverteilung vermahlen. Die Dispersion wurde in einem Zerstäubungstrockner bei 120°C Lufteingangstemperatur sprühgetrocknet und durch Zusatz von 10 g des obengenannten Dispergiermittels auf die Endfarbstärke eingestellt.
   Das so erhaltene Farbstoffpulver weist die in der Naßstufe erzielte Feinverteilung auf und eignet sich sehr gut zum Färben von Polyesterfasern und Mischgeweben aus Polyeester und Baumwolle.
   Insbesondere beim Färben von Wickelkörpern aus texturierten Polyesterfasern wurden keinerlei Abfiltrationen oder Unegalitäten festgestellt.

### Beispiel 2

Man diazotierte 226 g 4-Amino-2',4'-dimethylbenzophenon analog Beispiel 1. Zur Kupplungsreaktion legte man jedoch eine Lösung von 164 g 1,4-Dimethyl-5-cyano-2-hydroxypyrid-6-on und 50 g Natriumacetat in 2000 ml Wasser (pH-Wert ca. 4) vor. Man ließ nun das Diazoniumsalzgemisch so zum Pyridon laufen, daß der pH-Wert des resultierenden Gemisches stets im Bereich von 3,5 bis 5,5 blieb.

Die Reaktion war rasch beendet. Der ausgefallene Farbstoff schmilzt bei ca. 70°C und kann deshalb nicht durch Tempern in eine hochschmelzende Form gebracht werden. Wenn man jedoch den pH-Wert des entstandenen Reaktionsgemisches mit Natronlauge auf 8 stellt und die Suspension 4 Stunden bei Raumtemperatur nachrührt, erhält man nach Tempern, wie in Beispiel 1 beschrieben, die hochschmelzende Form.

### Beispiel 3

Ein Gemisch aus 127 g m-Xylol (wasserfrei) und 186 g p-Nitrobenzoylchlorid wurde mit 3 g wasserfreiem Eisen(III)chlorid versetzt und dann auf 90 bis 100°C erhitzt, wobei Chlorwasserstoffentwicklung auftrat. Man rührte 5 Stunden bei 90 bis 100°C nach. Dann wurde die Schmelze auf 450 ml Wasser ausgerührt und der Überschuß an m-Xylol mittels Wasserdampfdestillation entfernt. Man gab 1 g eines sauer wirksamen Netzmittels hinzu und kühlte das Gemisch auf Raumtemperatur ab, so daß 4-Nitro-2',4'-dimethylbenzophenon kristallin ausfiel. Nach Absaugen, Waschen und Trocknen erhielt man 253 g des Benzophenons der Formel 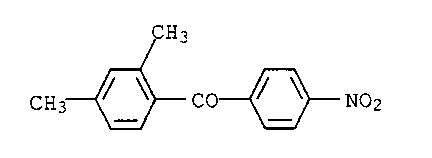
in reiner Form.

## Patentansprüche (Patentansprüche für folgende(n) Vertragsstaat(en): CH, DE, FR, GB, IT, LI)

1. Verfahren zur Herstellung von Nitrobenzophenonen der Formel I 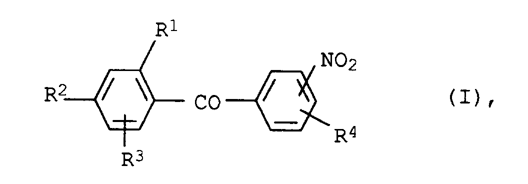 in der R¹ und R² gleich oder verschieden sind und unabhängig voneinander jeweils C₁-C₄-Alkyl, R³ Wasserstoff und R⁴ Wasserstoff oder Halogen bedeuten, durch Umsetzung von Aromaten der Formel II 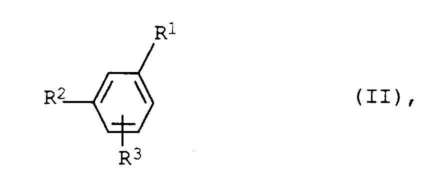 in der R¹, R² und R³ jeweils die obengenannte Bedeutung besitzen, mit Nitrobenzoylchloriden der Formel III 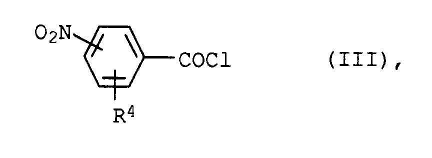 in der R⁴ die obengenannte Bedeutung besitzt, in Gegenwart einer Lewis-Säure, dadurch gekennzeichnet, daß man die Umsetzung im wesentlichen in Abwesenheit von inerten Lösungsmitteln bei einer Temperatur von 50 bis 110°C und einem Molverhältnis von Aromat der Formel II : Nitrobenzoylchlorid III von 1 : 1 bis 2 : 1 durchführt und als Lewis-Säure katalytische Mengen an Eisen(III)chlorid verwendet.

2. Verfahren nach Anspruch 1, dadurch gekennzeichnet, daß man die Umsetzung bei einem Molverhältnis von Aromat II : Nitrobenzoylchlorid III von 1,1 : 1 bis 1,3 : 1 durchführt.

3. Verfahren nach Anspruch 1, dadurch gekennzeichnet, daß R¹ und R² jeweils Methyl und R³ Wasserstoff bedeuten.

4. Verfahren nach Anspruch 1, dadurch gekennzeichnet, daß man p-Nitrobenzoylchlorid umsetzt.

5. Färbestabile Modifikation des Farbstoffs der Formel IV 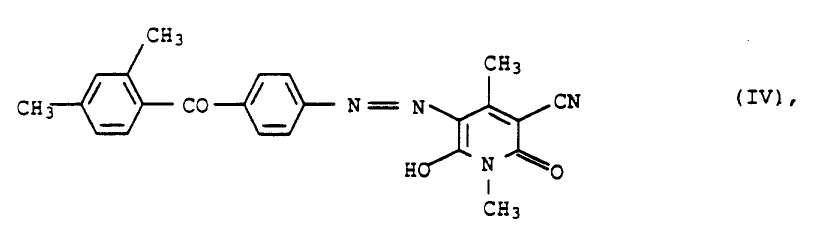 mit dem Röntgenbeugungsdiagramm (Cu-K_{α}-Strahlung) mit folgenden Linien starker Intensität bei Beugungswinkel Θ [°] von 8,085, 12,772, 13,644, 24,864 und 25,656, Linien mittlerer Intensität bei Beugungswinkel Θ [°] von 16,402, 18,894, 20,822, 21,940, 27,546 und 28,256 sowie Linien schwacher Intensität bei Beugungswinkel Θ [°] von 14,490, 15,352 und 23,711.

6. Verwendung der färbestabilen Modifikation des Farbstoffs gemäß Anspruch 5 zum Färben oder Bedrucken von synthetischem Fasermaterial.

## Patentansprüche (Patentansprüche für folgende(n) Vertragsstaat(en): ES)

1. Verfahren zur Herstellung von Nitrobenzophenonen der Formel I 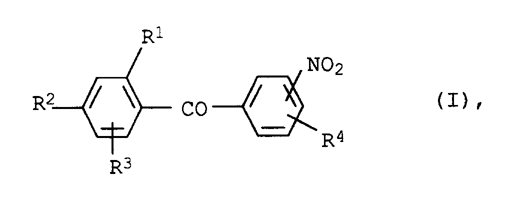 in der R¹ und R² gleich oder verschieden sind und unabhängig voneinander jeweils C₁-C₄-Alkyl, R³ Wasserstoff und R⁴ Wasserstoff oder Halogen bedeuten, durch Umsetzung von Aromaten der Formel II 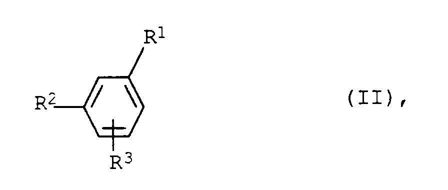 in der R¹, R² und R³ jeweils die obengenannte Bedeutung besitzen, mit Nitrobenzoylchloriden der Formel III 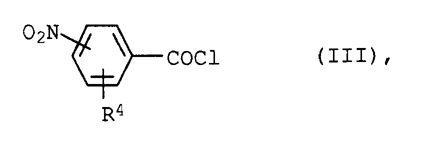 in der R⁴ die obengenannte Bedeutung besitzt, in Gegenwart einer Lewis-Säure, dadurch gekennzeichnet, daß man die Umsetzung im wesentlichen in Abwesenheit von inerten Lösungsmitteln bei einer Temperatur von 50 bis 110°C und einem Molverhältnis von Aromat der Formel II : Nitrobenzoylchlorid III von 1 : 1 bis 2 : 1 durchführt und als Lewis-Säure katalytische Mengen an Eisen(III)chlorid verwendet.

2. Verfahren nach Anspruch 1, dadurch gekennzeichnet, daß man die Umsetzung bei einem Molverhältnis von Aromat II : Nitrobenzoylchlorid III von 1,1 : 1 bis 1,3 : 1 durchführt.

3. Verfahren nach Anspruch 1, dadurch gekennzeichnet, daß R¹ und R² jeweils Methyl und R³ Wasserstoff bedeuten.

4. Verfahren nach Anspruch 1, dadurch gekennzeichnet, daß man p-Nitrobenzoylchlorid umsetzt.

5. Verfahren zur Herstellung einer färbestabilen Modifikation des Farbstoffs der Formel IV 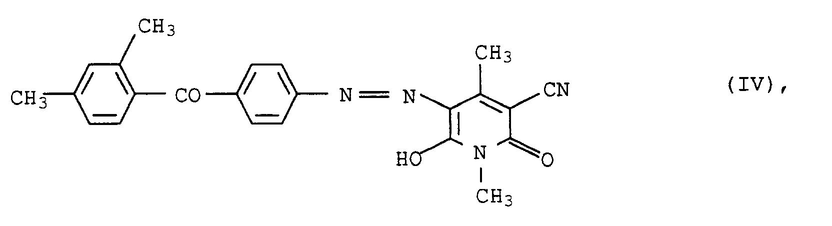 mit dem Röntgenbeugungsdiagramm (Cu-K_{α}-Strahlung) mit folgenden Linien starker Intensität bei Beugungswinkel Θ [°] von 8,085, 12,772, 13,644, 24,864 und 25,656, Linien mittlerer Intensität bei Beugungswinkel Θ [°] von 16,402, 18,894, 20,822, 21,940, 27,546 und 28,256 sowie Linien schwacher Intensität bei Beugungswinkel Θ [°] von 14,490, 15,352 und 23,711, dadurch gekennzeichnet, daß man den Farbstoff der Formel IV nach seiner Herstellung 0,5 bis 4 Stunden in wäßriger Lauge bei einem pH-Wert von 7 bis 9 und bei einer Temperatur von 20 bis 90°C behandelt.

6. Verwendung der färbestabilen Modifikation des Farbstoffs gemäß Anspruch 5 zum Färben oder Bedrucken von synthetischem Fasermaterial.

## Claims (Claims for the following Contracting State(s): CH, DE, FR, GB, IT, LI)

1. A process for preparing nitrobenzophenones of the formula I 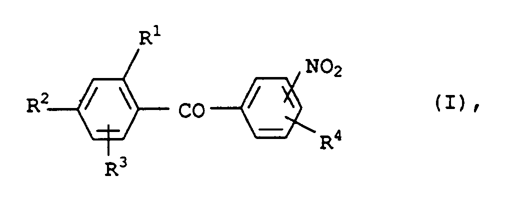 where R¹ and R² are identical or different and each, independently of one another, is C₁-C₄-alkyl, R³ is hydrogen and R⁴ is hydrogen or halogen, by reacting aromatic compounds of the formula II 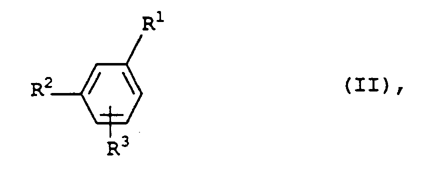 where R¹, R² and R³ each have the abovementioned meanings, with nitrobenzoyl chlorides of the formula III 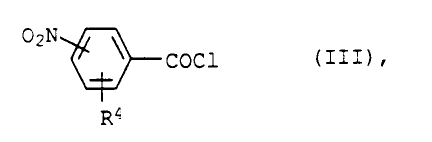 where R⁴ has the abovementioned meanings, in the presence of a Lewis acid, wherein the reaction is carried out essentially in the absence of inert solvents at from 50 to 110°C and with a molar ratio of aromatic compound of the formula II to nitrobenzoyl chloride III of from 1:1 to 2:1, and catalytic amounts of iron(III) chloride are used as Lewis acid.

2. A process as claimed in claim 1, wherein the reaction is carried out with a molar ratio of aromatic compound II to nitrobenzoyl chloride III of from 1.1:1 to 1.3:1.

3. A process as claimed in claim 1, wherein R¹ and R² are each methyl and R³ is hydrogen.

4. A process as claimed in claim 1, wherein p-nitrobenzoyl chloride is reacted.

5. A color-stable modification of the dye of the formula IV 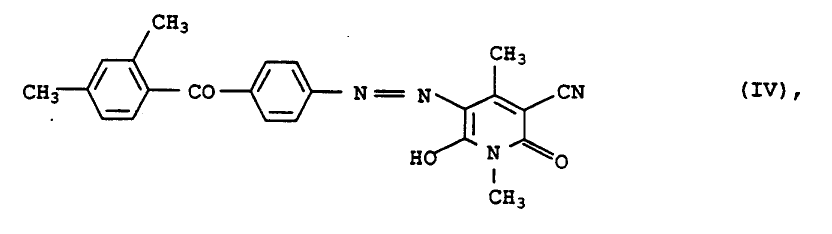 whose X-ray diffraction diagram (Cu-K_{α} radiation) has the following lines of strong intensity at diffraction angles θ [°] of 8.085, 12.772, 13.644, 24.864 and 25.656, lines of moderate intensity at diffraction angles θ [°] of 16.402, 18.894, 20.822, 21.940, 27.546 and 28.256, and lines of weak intensity at diffraction angles θ [°] of 14.490, 15.352 and 23.711.

6. The use of the color-stable modification of the dye as claimed in claim 5 for dyeing or printing synthetic fiber material.

## Claims (Claims for the following Contracting State(s): ES)

1. A process for preparing nitrobenzophenones of the formula I 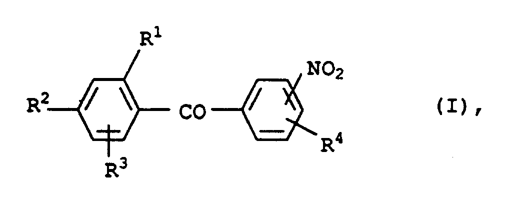 where R¹ and R² are identical or different and each, independently of one another, is C₁-C₄-alkyl, R³ is hydrogen and R⁴ is hydrogen or halogen, by reacting aromatic compounds of the formula II 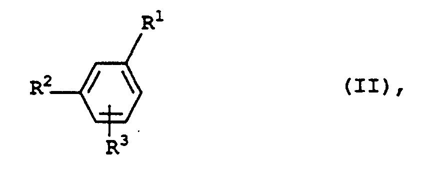 where R¹, R² and R³ each have the abovementioned meanings, with nitrobenzoyl chlorides of the formula III 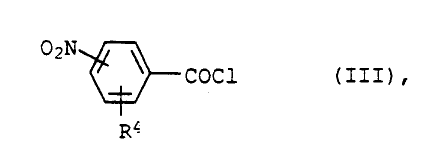 where R⁴ has the abovementioned meanings, in the presence of a Lewis acid, wherein the reaction is carried out essentially in the absence of inert solvents at from 50 to 110°C and with a molar ratio of aromatic compound of the formula II to nitrobenzoyl chloride III of from 1:1 to 2:1, and catalytic amounts of iron(III) chloride are used as Lewis acid.

2. A process as claimed in claim 1, wherein the reaction is carried out with a molar ratio of aromatic compound II to nitrobenzoyl chloride III of from 1.1:1 to 1.3:1.

3. A process as claimed in claim 1, wherein R¹ and R² are each methyl and R³ is hydrogen.

4. A process as claimed in claim 1, wherein p-nitrobenzoyl chloride is reacted.

5. A process for preparing a color-stable modification of the dye of the formula IV 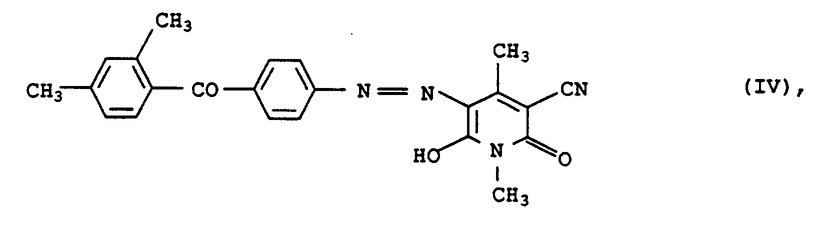 whose X-ray diffraction diagram (Cu-K_{α} radiation) has the following lines of strong intensity at diffraction angles θ [°] of 8.085, 12.772, 13.644, 24.864 and 25.656, lines of moderate intensity at diffraction angles θ [°] of 16.402, 18.894, 20.822, 21.940, 27.546 and 28.256, and lines of weak intensity at diffraction angles θ [°] of 14.490, 15.352 and 23.711 wherein, after its preparation, the dye of the formula IV is treated with aqueous alkali at pH 7-9 and at from 20 to 90°C for from 0.5 to 4 hours.

6. The use of the color-stable modification of the dye as claimed in claim 5 for dyeing or printing synthetic fiber material.

## Revendications (Revendications pour l'(les) Etat(s) contractant(s) suivant(s): CH, DE, FR, GB, IT, LI)

1. Procédé de préparation de nitrobenzopbénones de formule I 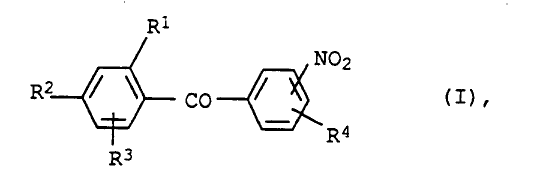 dans laquelle R¹ et R² sont identiques ou différente et représentent chacun, indépendamment l'un de l'autre, un groupement alkyle en C₁-C₄, R³ représente un atome d'hydrogène et R⁴ représente un atome d'hydrogène ou d'halogène, par réaction de composés aromatiques de formule II 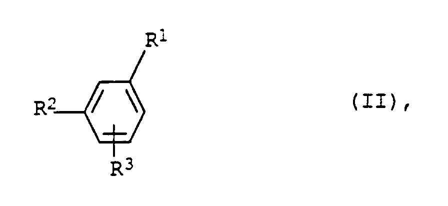 dans laquelle R¹, R² et R³ ont chacun la signification donnée ci-dessus, avec des chlorures de nitrobenzoyle de formule III 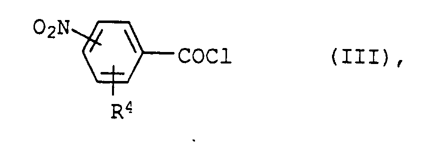 dans laquelle R⁴ a la signification donnée ci-dessus, en présence d'un acide de Lewis, caractérisé en ce que l'on conduit essentiellement la réaction en l'absence de solvants inertes, à une température de 50 à 110°C et avec un rapport molaire du composé aromatique de formule II au chlorure de nitrobenzoyle III de 1:1 à 2:1, et on utilise, comme acide de Lewis, des quantités catalytiques de chlorure ferrique.

2. Procédé selon la revendication 1, caractérisé en ce que l'on conduit la réaction avec un rapport molaire du composé aromatique II au chlorure de nitrobenzoyle III de 1,1 : 1 à 1,3 : 1.

3. Procédé selon la revendication 1, caractérisé en ce que R¹ et R² représentent chacun un groupement méthyle et R³ représente un atome d'hydrogène.

4. Procédé selon la revendication 1, caractérisé en ce que l'on met en réaction du chlorure de p-nitrobenzoyle.

5. Variété de couleur stable du colorant de formule IV 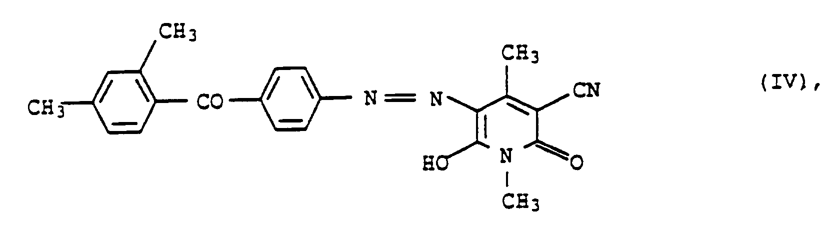 ayant le diagramme de diffraction des rayons X (rayonnement Cu-K_{α}) qui présente successivement des lignes de forte intensité aux angles de diffraction θ (°) de 8,085, 12,772, 13,644, 24,864 et 25,656 , des lignes de moyenne intensité aux angles de diffraction θ (°) de 16,402, 18,894, 20,822, 21,940, 27,546 et 28,256, ainsi que des lignes de faible intensité aux angles de diffraction θ (°) de 14,490, 15,352 et 23,711.

6. Utilisation de la variété de couleur stable du colorant selon la revendication 5 pour la teinture ou l'impression de matières en fibres synthétiques.

## Revendications (Revendications pour l'(les) Etat(s) contractant(s) suivant(s): ES)

1. Procédé de préparation de nitrobenzophénones de formule I 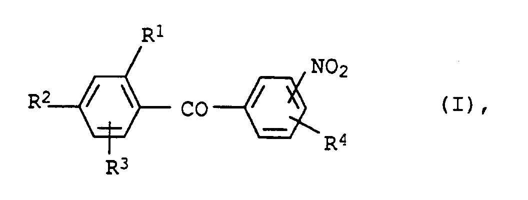 dans laquelle R¹ et R² sont identiques ou différents et représentent chacun, indépendamment l'un de l'autre, un groupement alkyle en C₁-C₄, R³ représente un atome d'hydrogène et R⁴ représente un atome d'hydrogène ou d'halogéne, par réaction de composés aromatiques de formule II 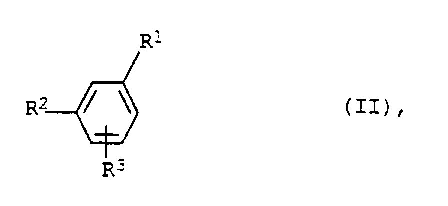 dans laquelle R¹, R² et R³ ont chacun la signification donnée ci-dessus, avec des chlorures de nitrobenzoyle de formule III 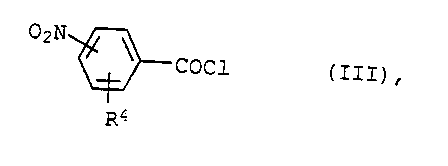 dans laquelle R⁴ a la signification donnée ci-dessus, en présence d'un acide de Lewis, caractérisé en ce que l'on conduit essentiellement la réaction en l'absence de solvants inertes, a une température de 50 à 110°C et avec un rapport molaire du composé aromatique de formule II au chlorure de nitrobenzoyle III de 1:1 à 2:1, et on utilise, comme acide de Lewis, des quantités catalytiques de chlorure ferrique.

2. Procédé selon la revendication 1, caractérisé en ce que l'on conduit la réaction avec un rapport molaire du composé aromatique II au chlorure de nitrobenzoyle III de 1,1 : 1 à 1,3 : 1.

3. Procédé selon la revendication 1, caractérisé en ce que R¹ et R² représentent chacun un groupement méthyle et R³ représente un atome d'hydrogène.

4. Procédé selon la revendication 1, caractérisé en ce que l'on met en réaction du chlorure de p-nitrobenzoyle.

5. Procédé de préparation d'une variété de couleur stable du colorant de formule IV 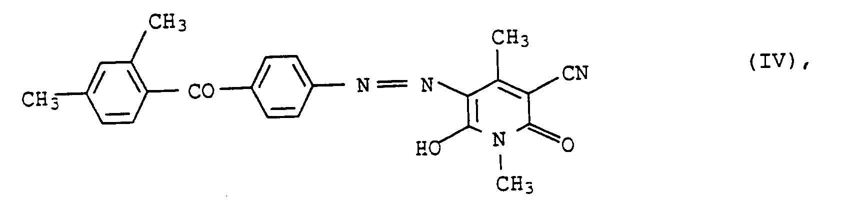 ayant le diagramme de diffraction des rayons X (rayonnement Cu-K_{α}) qui présente successivement des lignes de forte intensité aux angles de diffraction θ (°) de 8,085, 12,772, 13,644, 24,864 et 25,656 , des lignes de moyenne intensité aux angles de diffraction θ (°) de 16,402, 18,894, 20,822, 21,940, 27,546 et 28,256, ainsi que des lignes de faible intensité aux angles de diffraction θ (°) de 14,490, 15,352 et 23,711, caractérisé en ce que l'on traite le colorant de formule IV, après sa préparaion, pendant 0,5 à 4 h, dans de la lessive alcaline aqueuse à un pH de 7 à 9 et à une température de 20 à 90°C.

6. Utilisation de la variété de couleur stable du colorant selon la revendication 5 pour la teinture ou l'impression de matières en fibres synthétiques.
